# EUROPEAN PATENT APPLICATION

(11) **EP 1 779 848 A1**
(43) Date of publication of application: **02.05.2007**
(21) Application number: 05110149.1
(22) Date of filing: 28.10.2005
(51) Int. Cl.: A61K 31/365, A61K 31/7048, A61K 31/4015, A61K 31/4166, A61K 38/15, A61P 17/06, A61P 19/02, A61P 37/00, A61P 1/00

(54) **V-ATPase inhibitors for the treatment of inflammatory and autoimmune diseases**

(71) Applicant: NIKEM RESEARCH S.R.L., 20021 Baranzate di Bollate (IT)
(72) Inventor: FARINA, Carlo, 20021, BARANZATE DI BOLLATE (IT); CONSTANTIN, Gabriela, 37020, S. FLORIANO (IT); LAUDANNA, Carlo, 37020, S. FLORIANO (IT); MISIANO, Paola, 20021, BARANZATE DI BOLLATE (IT)
(74) Representative: Gerli, Paolo

(57) **Abstract**

The use of vacuolar ATPase inhibitors in the prevention and/or treatment of inflammatory and autoimmune diseases is herein described. Vacuolar ATPase inhibitors are selected among products of natural, semi-synthetic or synthetic origin; more preferred are inhibitors based on a 2- and/or 3-substituted indole, azaindole or benzimidazole structure.

## Description

### FIELD OF THE INVENTION

The present invention relates to the filed of the medical treatment of inflammatory and autoimmune disorders.

### STATE OF THE ART

Inflammation is the effector phase of the immune response. It can be viewed as a homeostatic process capable of developing a stereotyped reaction to cell and tissue damages. The main role of the inflammatory response consists in protecting the organism for infectious disease and in repairing wounds derived form tissue injuries.

The inflammatory response is closely intertwined with the process of repair. Inflammation serves to destroy, dilute, or wall off the injurious agent, and it sets into motion a series of events that try to heal and reconstitute the damaged tissue. Repair begins during the early phases of inflammation but reaches completion usually after the injurious influence has been neutralized. During repair, the injured tissue is replaced through regeneration of native parenchymal cells, by filling of the defect with fibrous tissue or, most commonly, by a combination of these two processes.

Inflammation is fundamentally a protective response and, indeed, without inflammation, infections would go unchecked, wounds would never heal, and injured organs might remain permanent festering sores. However, inflammation and repair may be potentially harmful. Inflammatory reactions, for example, underlie common chronic diseases, such as rheumatoid arthritis, atherosclerosis, multiple sclerosis and lung fibrosis, as well as life-threatening hypersensitivity (anaphylaxis) reactions to insect bites, drugs, and toxins. Repair by fibrosis may lead to disfiguring scars or fibrous bands that cause intestinal obstruction or limit the mobility of joints.

The inflammatory response consists of two main components, a vascular reaction and a cellular reaction. Many tissues and cells are involved in these reactions, including the fluid and proteins of plasma, circulating cells, blood vessels, and cellular and extracellular constituents of connective tissue. The circulating cells include neutrophils, monocytes, eosinophils, lymphocytes, basophils, and platelets. The connective tissue cells are the mast cells, which intimately surround blood vessels, the fibroblasts, resident macrophages and lymphocytes. The extracellular matrix consists of the structural fibrous proteins (collagen, elastin), adhesive glycoproteins (like fibronectin, laminin, nonfibrillar collagen and tenascin), and proteoglycans. The basement membrane is a specialized component of the extracellular matrix consisting of adhesive glycoproteins and proteoglycans.

Inflammation is divided into acute and chronic patterns. Acute inflammation is rapid in onset (seconds or minutes) and is of relatively short duration, lasting for minutes, several hours, or a few days; its main characteristics are the exudation of fluid and plasma proteins (edema) and the emigration of leukocytes, predominantly neutrophils. Chronic inflammation is of longer duration and is associated histologically with the presence of lymphocytes and macrophages, the proliferation of blood vessels, fibrosis, and tissue necrosis. Many factors modify the course and morphologic appearance of both acute and chronic inflammation.

The vascular and cellular reactions of both acute and chronic inflammation are mediated by chemical factors that are derived from plasma proteins or cells and are produced in response to or activated by the inflammatory stimulus. Such mediators, acting singly, in combinations, or in sequence, then amplify the inflammatory response and influence its evolution.

Inflammation is terminated when the offending agent is eliminated and the secreted mediators are broken down or dissipated. However, during chronic inflammatory disease, such as autoimmune diseases, the self containing and limiting capability of inflammation is lost and the process proceed out of control leading to consistent and permanent damage. Controlling the evolution and limiting the intensity of the inflammatory response during chronic inflammatory diseases as well as during some diseases associated with acute inflammation is one of the major goal of the pharmacological research today.

Autoimmune diseases are a family of more than 80 chronic, and often disabling, illnesses. They include among other autoimmune diseases: type 1 diabetes, systemic lupus erythematosus, multiple sclerosis, rheumatoid arthritis, inflammatory bowel diseases, including both Crohn's disease and ulcerative colitis, hemolytic anemia, Graves' disease, scleroderma, psoriasis, autoimmune thyroid diseases, Sjögren's syndrome, eye autoimmune diseases, myasthenia gravis, Guillain-Barre'syndrome, Addison's disease (www.niaid.nih.gov/dait/pdf/ADCC_Report;www.cureautoimmunity.org).

Autoimmune diseases occur in up to 3-5% of the general population causing significant and chronic morbidity and disability *(*Marrack P., et al. Nature Medicine 7: 899-905, 2001*).* For instance, they affect 14.7 to 23.5 million people in the US country, and their prevalence is rising. Many of these diseases are classified according to what organs and tissues are targeted by the damaging immune responses. There is an autoimmune disease specific for nearly every organ in the body, involving, usually, response to an antigen expressed only in that organ. Immune-mediated injuries localized to a single organ or tissue, such as the pancreas in type 1 diabetes and the central nervous system in multiple sclerosis, characterize organ-specific autoimmune diseases. In contrast, non-organ-specific diseases, such as systemic lupus erythematosus, are characterized by immune reactions against many different organs and tissues resulting in widespread injury.

Autoimmune diseases are chronic inflammatory diseases controlled by host genes and the environment. Both can increase susceptibility to autoimmunity by affecting the overall reactivity and quality of the cells of the immune system. Antigen/organ specificity is affected by antigen presentation and recognition, antigen expression and the state and response of the target organs.

Multiple sclerosis (MS) is considered a neurological disease caused by an autoimmune attack directed against brain antigens. Prevalence of MS is 1-in-700 in USA (NIAID, www.niaid.nih.gov) and 1-in-1100 in Italy (www.aism.it). About 9.600 new MS cases are diagnosed each year in USA and about 1.800 new MS cases are diagnosed in Italy. In most patients (85%), MS begins as a relapsing illness with episodes of neurological dysfunction lasting several weeks, followed by substantial or complete improvement (relapsing-remitting MS). However, with time and repeated relapses, recovery is often less complete, and a gradual clinical progression develops (secondary progressive MS). In a small proportion of patients (15%), the decline in neurological dysfunction is gradual, beginning with the onset of the disease (primary progressive MS) *(*Noseworthy, J.H., Nature, 399, A40-7, 1999). Autoimmune brain damage is mediated in MS by T cells, and both CD4⁺ and CD8⁺ T cells seems to have crucial roles. Pathogenic lymphocytes egress from the blood stream across the blood brain barrier and exert their pathological effect which leads to damage to glia and neurons. Leukocyte adhesion to brain endothelium and then transmigration into the brain parenchima represent crucial events in the pathogenesis of MS and its animal model experimental autoimmune encephalomyelitis (EAE) (Hickey W.F., Brain Pathol., 1(2):97-105,1991). Adhesion to vascular endothelium and transmigration of activated lymphocytes into the target organ represent crucial events in the pathogenesis of autoimmune diseases leading to chronic inflammation and tissue destruction.

There is a clear paucity of drugs that successfully treat chronic autoimmune diseases *(*Feeldman M. et al., Nature. 2, 435, 612-9, 2005*).* For instance, as shown for other autoimmune diseases, the current drugs that are widely prescribed for MS have significant limitations, including cost (approx 11.000 $ per year), inconvenience (parenteral administration), frequency of adverse effects (especially flu-like symptoms for several hours in many patients after each injection of interferon) and a relatively modest impact on disease course.

The vacuolar proton translocating ATPases (V-ATPases) are widely expressed in eucaryotic cell endomembranes where protons are pumped into the organelle lumen using the energy released by ATP hydrolysis. The active transport of protons across animal cell plasma membranes appears to be restricted to cells that are specialized in proton secretion such as macrophages (J.Biol.Chem., 265,7645, 1990*),* and the osteoclasts *(*J.Cell.Biol., 105, 1637, 1987*).* In these cells, V-ATPase actively secretes protons from the cells and establishes an acidic extracellular environment useful respectively for bacteria phagocytosis, urinary acidification, and bone resorption. The inhibition of V-ATPase has recently been identified as a possible therapeutic target. In particular, V-ATPase inhibitors have been described for use in preventing bone loss and inhibiting bone resorption, thereby being useful in the treatment of osteoporosis and other conditions related to osteoclast hyperactivity (Acta Physiol.Scand., , 163(suppl.), 195, 1998*.;* J.Clin.Invest., , 106, 309, 2000). A number of V-ATPase inhibitors useful for the treatment of osteoclast-related disorders and/or tumours have been the subject of a number of the patent applications US2002099080, W09801443, W00100587, W00102388, PCT/EP2005/051908, PCT/EP2005/051910.

### SUMMARY

We have now discovered that V-ATPase inhibitors have immunomodulatory effects on cells of innate and acquired immunity, together with anti-inflammatory properties.

In the present application we describe a potent inhibitory activity of these compounds on adhesion, chemotaxis and free radicals release of immunocompetent cells; furthermore, the outstanding activity on the in vivo animal model of multiple sclerosis, the experimental autoimmune encephalomyelitis (EAE), strongly support the therapeutic potential of these compounds. These compounds, can therefore be utilized in the treatment and/or prevention of acute and chronic inflammatory diseases and autoimmune diseases, and in the treatment of septic shock.

### DESCRIPTION OF THE FIGURES

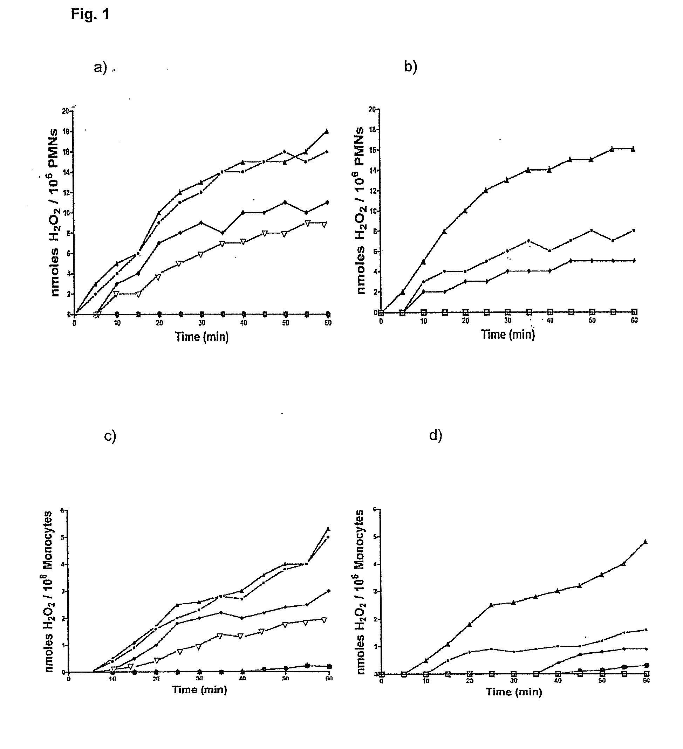

**Figure 1**: Effect of V-ATPase inhibitors on NADPH-oxidase activation
   **a)** Effect of Compound of Example 1 (PCT/EP2005/051908) in PMNs:
      -▲- :Control with PMA;
      -•- :10 µM Compound of Example 1 with PMA;
      -◆- :50 µM Compound of Example 1 with PMA;
      - - :100 µM Compound of Example 1 with PMA;
      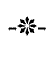 Control without PMA
   **b)** Effect of Compound of Example 2 (PCT/EP2005/051910) in PMNs:
      -▲-:Control with PMA;
      -•- :1 µM Compound of Example 2 with PMA;
      -◆- :5 µM Compound of Example 2 with PMA;
      -□- :10 µM Compound of Example 2 with PMA, and control without PMA (n.b.: both these curves overlap in figure 1 b)
   **c)**Effect of Compound of Example 1 (PCT/EP2005/051908) in Monocytes:
      -▲-: Control with PMA;
      -•-:10 µM Compound of Example 1 with PMA;
      -◆-:50 µM Compound of Example 1 with PMA;
      - -:100 µM Compound of Example 1 with PMA;
      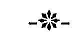 : Control without PMA
   **d)**Effect of Compound of Example 2 (PCT/EP2005/051910) in Monocytes:
      -▲-: Control with PMA;
      -•-:1 µM Compound of Example 2 with PMA;
      -◆-:5 µM Compound of Example 2 with PMA;
      -□-:10 µM Compound of Example 2 with PMA;
      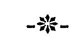 : Control without PMA;
      (n.b.: the curves -◆-, -□- and -*- partly overlap in figure 1d.)

### DETAILED DESCRIPTION OF THE INVENTION

Object of the present invention is the use of one or more V-ATPase inhibitors in the preparation of a medicament useful for preventing and/or treating inflammatory and autoimmune diseases.

The invention also comprises a method for preventing and/or treating inflammatory and autoimmune diseases, characterised by the administration of a pharmaceutically effective amount of a compound of formula (I) to a patient in need thereof.

The prevention and/or treatment of septic shock by use of said V-ATPase inhibitors is object of co-pending application in the name of the present Applicant.

V-ATPase inhibitors are a recognised class of compounds, as reviewed in Curr.Pharm.Design,,8,2033-2048, 2002*.* In general, for V-ATPase inhibitor is meant a compound which, when assayed according to James I.E. et al. - J. Bone Min. Res. 14, 1562-1569, 1999 and Nadler, G. et al.- Bioorg. Med. Chem. Letters 8, 3621-3626, 1998, shows at least 50% Bafilomycin-sensitive inhibitory activity at 5 µM.

V-ATPase inhibitors may be of natural origin, semi-synthetic or entirely synthetic.

Non limiting examples of V-ATPase inhibitors of natural origin are the macrolides bafilomycins, concanamycins, depsipeptide mycotoxins derived from the fungi *Metharisium anisopliae*, destruxin, lobatamides, salicylihalamides, oximidines; non-limiting examples of semi-synthetic derivatives are the sulphonamide derivatives of bafilomycins, 7,21-0-disubstituted bafilomycins, 2-methoxy-2,4-pentadienoic esters of bafilomycins, etc.; non-limiting examples of entirely synthetic V-ATPase inhibitors are N-ethylmaleimide, 7-chloro-4-nitrobenzo-2-oxa-1,3-diazole, and derivatives thereof, the compounds WY 47766, SB242784, aminoquinoline derivatives like the compound FR167356. A particularly preferred class of V-ATPase inhibitors is that of 2- and/or 3- substituted indoles, azaindoles, benzimidazoles; within this class, even more preferred are the inhibitors compounds disclosed in the following patent applications, all of which are incorporated herein by reference.
(i) US2002099080, describing compounds of formula 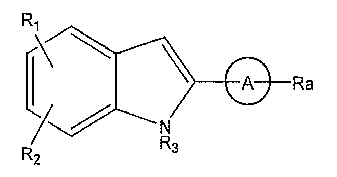 wherein A, R1-3, Ra have the meanings therein indicated.
(ii) W09801443, describing compounds of formula 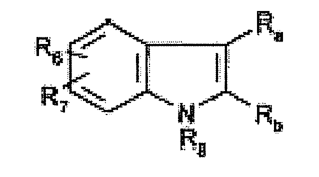 wherein R6-8, Ra, Rb have the meanings therein indicated.
(iii) W001 00587, describing compounds of formula: 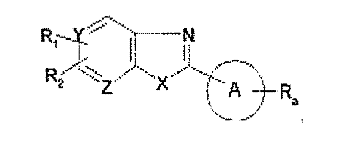 wherein R1, R2, A, Ra, X,Y,Z have the meanings therein indicated.
(iv) W00102388, describing compounds of formula 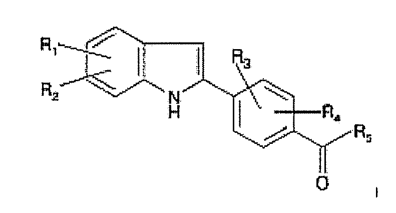 wherein R1-R5 have the meanings therein indicated.
(v) PCT/EP2005/051908, describing compounds of formula: 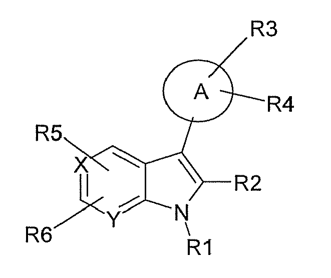 wherein:
   R1 is chosen from H, alkyl, arylalkyl, hydroxyalkyl, alkoxyalkyl, aminoalkyl, alkylaminoalkyl, dialkylaminoalkyl, alkylCOOalkyl, alkoxycarbonylalkyl, aminocarbonylalkyl, alkylaminocarbonylalkyl, dialkylaminocarbonylalkyl, alkylCONalkyl, cyanoalkyl, or a group R'R"Nalkyl, in which R' and R", together with the nitrogen atom to which they are attached, may form a 5, 6 or 7 membered ring, optionally containing a heteroatom chosen from O, S and N, and where said N atom may be substituted by alkyl;
   R2 is chosen from alkyl, alkenyl, aryl, heterocyclyl optionally substituted by alkyl or aryl, acid, ester, amide, nitrile, aminoalkyl, alkylaminoalkyl, dialkylaminoalkyl, hydroxyalkyl, CH₂NHCOCH₃, CONHSO₂CH₃, alkoxycarbonylalkyl, alkoxycarbonylalkenyl; or R1 and R2 together form a 5, 6 or 7 membered ring containing optionally a heteroatom chosen from O, S, N and containing optionally a carbonyl function which can be attached to any carbon atom of said ring, and where said N atom may be substituted by alkyl, aryl, arylalkyl, heteroaryl, alkylsulfonyl, arylsulfonyl, alkylcarbonyl, arylcarbonyl, alkylaminocarbonyl, arylaminocarbonyl;
   R3, R4, R5, R6 each independently represent H, alkyl, alkoxy, hydroxy, halogen, trifluoromethyl, trifluoromethyloxy;
   X and Y each independently represent carbon or nitrogen;
   A is chosen from a phenyl or a heterocyclic ring with 5 or 6 members containing up to two heteroatoms chosen from nitrogen, oxygen and sulfur.
(vi) PCT/EP2005/051910 describing compounds of formula: 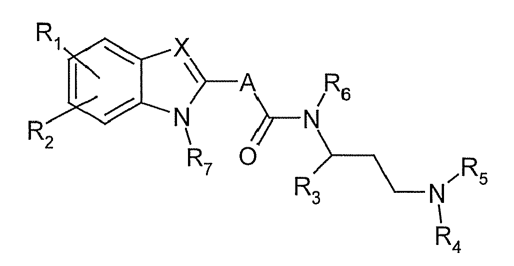 wherein:
   X is chosen from -CH- or -N-;
   A is chosen from the groups: 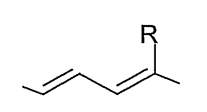 R= alkoxy, hydroxyalkoxy 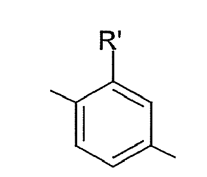 R'= hydroxy, alkoxy, hydroxyalkoxy, halogen
   R1 and R2, are each independently chosen from H, halogen and alkoxy;
   R3 and R4 are each independently chosen from H, alkyl, or R3 and R4, together with the atoms to which they are attached, form a 6, 7 or 8 membered heterocycle containing an atom of nitrogen, optionally substituted by one or more alkyl groups;
   R5 is chosen from H, alkyl, hydroxyalkyl, alkoxyalkyl, carboxyalkyl, aminoalkyl, optionally substituted aryl or arylalkyl, optionally substituted heterocyclyl or heterocyclylalkyl, or R5 and R4, together with the nitrogen atom to which they are attached, form an optionally substituted 5-8 membered heterocyclic ring containing up to 2 heteroatoms chosen from N, O and S;
   R6 and R7 are independently chosen from H and alkyl.

The present compounds can be suitably used in the treatment of inflammatory and autoimmune diseases.

Inflammatory diseases may be either acute or chronic; examples thereof are rheumatoid arthritis, atherosclerosis, multiple sclerosis, lung fibrosis, life-threatening hypersensitivity (anaphylaxis), reactions to insect bites, drugs, and toxins; chronic inflammatory bowel diseases, such as irritable bowel syndrome, colitis and other inflammatory diseases of the gastrointestinal tract; nephritis; inflammatory skin diseases like eczema, dermatitis, ichthyosis, acne, skin hypersensitivity reactions;

Examples of autoimmune diseases are type 1 diabetes, systemic lupus erythematosus, multiple sclerosis, rheumatoid arthritis, inflammatory bowel diseases, including both Crohn's disease and ulcerative colitis, hemolytic anemia, Graves' disease, scleroderma, autoimmune thyroid diseases, Sjögren's syndrome, psoriasis, eye autoimmune diseases, myasthenia gravis, Guillain-Barre' syndrome and Addison's disease.

The present V-ATPase inhibitors can be administered as such or preferably as in the form of pharmaceutical compositions in the presence of suitable pharmaceutical excipients. The dosage units of these pharmaceutical compositions may contain said inhibitors in a quantity between 1 and 1000 mg. The above compositions and dosage units are adapted to deliver to the patients effective amounts of V-ATPase inhibitor. Said inhibitors are generally active within a dosage range comprised between 0.01 and 100 mg/Kg.

The V-ATPase inhibitors can be administered alone or in association with further drugs suitable for joint therapy with antiinflammatory or immunosuppressing agents; these further drugs are selected according to rules well-known in the medical field, in function of the specific inflammatory or autoimmune condition to be treated.

The pharmaceutical compositions of the invention can be adapted for the various administration routes, and can be provided for example in the form of injectable solutions, solutions for infusion, solutions for inhalation, suspensions, emulsions, syrups, elixirs, drops, suppositories, possibly coated pills, hard or soft capsules, microcapsules, granules, dispersible powders etc.

The excipients contained in these compositions are those commonly used in pharmaceutical technology, and can be used in the manner and quantity commonly known to the expert of the art.

Solid administration forms, such as pills and capsules for oral administration, are normally supplied in dosage units. They contain conventional excipients such as binders, fillers, diluents, tabletting agents, lubricants, detergents, disintegrants, colorants and wetting agents and can be coated in accordance with methods well known in the art.

The fillers include for example cellulose, mannitol, lactose and similar agents. The disintegrants include starch, polyvinylpyrrolidone and starch derivatives such as sodium starch glycolate; the lubricants include, for example, magnesium stearate; the wetting agents include for example sodium lauryl sulfate.

These solid oral compositions can be prepared with conventional mixing, filling or tabletting methods. The mixing operations can be repeated to disperse the active agent in compositions containing large quantities of fillers. These are conventional operations.

The liquid preparations can be provided as such or in the form of a dry product to be reconstituted with water or with a suitable carrier at the time of use. These liquid preparations can contain conventional additives such as suspending agents, for example sorbitol, syrup, methylcellulose, gelatin, hydroxyethylcellulose, carboxymethylcellulose, aluminium stearate gel or hydrogenated edible fats, emulsifying agents, for example lecithin, sorbitan monooleate, or acacia; non aqueous carriers (which can include edible oil) for example almond oil, fractionated coconut oil, oily esters such a glycerin esters, propylene glycol or ethyl alcohol; preservatives, for example methyl or propyl p-hydroxybenzoate or sorbic acid and if desired, conventional flavours or colorants.

The oral formulations also include sustained release conventional formulations, such as enteric coated pills or granules.

For parenteral administration, fluid dosage units can be prepared containing the compound and a sterile carrier. The compound, depending on the carrier and concentration, can be suspended or dissolved. The parenteral solutions are normally prepared by dissolving the compound in a carrier and sterilizing by filtration, before filling suitable vials or ampoules and sealing. Adjuvants such as local anaesthetics, preservatives and buffering agents can be advantageously dissolved in the carrier. In order to increase stability, the composition can be frozen after filling the vial and the water removed under vacuum. The parenteral suspensions are prepared essentially in the same way, with the difference that the compound can be suspended rather than dissolved in the carrier, and can be sterilized by exposure to ethylene oxide prior to being suspended in the sterile carrier. A surfactant or humectant can be advantageously included in the composition to facilitate uniform distribution of the compound of the invention.

As is the common practise, the compositions are normally accompanied by written or printed instructions, for use in the treatment concerned.

The invention is now illustrated by the following non-limiting examples.

### EXPERIMENTAL PART

### Methods

### In vitro studies

### Chemokine-induced adhesion in PMNs, lymphocytes and monocytes

Adhesion was performed on purified integrin ligands and was evaluated as reported in Constantin G. et al., Immunity, 16, 759-769, 2000*.* Briefly, primary naïve lymphocytes, polymorphonuclear neutrophils (PMNs) or monocytes were isolated form healthy donors. In other experiments, murine autoreactive T cells were obtained from SJL mice immunized with PLP139-151 as described *(*Piccio L. et al., J. Immunol. 168, 1940-1949, 2002*).* Cells were resuspended at 4x10⁶/ml in PBS, CaCl₂, MgCl₂ 1 mM, 10% FCS, pH 7.2. Adhesion assays were performed on eighteen well glass slides coated overnight at 4°C with ligands for CR3 (fibrinogen) or LFA-1 (ICAM-1). 20 µl of cell suspension were added to the wells and stimulated at 37°C with 5 µl of agonists prior to washing, fixation and computer-assisted enumeration of bound cells.

Integrin activation was measured as induction of rapid adhesion triggered by the classical chemoattractant N-formyl-L-methionyl-L-leucyl-phenylalanine (fMLP) (100 nM) or by the CC chemokine MIP3β (CCL19, 1 µM). PMNs and monocytes were triggered to adhere to fibrinogen (CR3 ligand); naive human lymphocytes or murine encephalitogenic T cells were triggered to adhere to ICAM-1 (LFA-1 ligand). Triggered adhesion was evaluated after 2 min of stimulation under static conditions. The cells were pretreated at 37°C for 30-60 min with the indicated concentrations of the test compound. Results are expressed as percentage of inhibition over control (= 100%) and are the mean of three experiments in the case of human leukocytes. Results are expressed as mean counts and are the mean counts ± S.D of three experiments and are the mean of three experiments in the case of murine encephalitogenic lymphocytes.

### Spontaneous adhesion of encephalitogenic T cells

Spontaneous adhesion of encephalitogenic lymphocytes was measured in adhesion assays on purified ICAM-1 and VCAM-1 as previously described *(*Constantin G. et al., J. Immunol. 162, 1144-1149, 1999). Autoreactive T cells were obtained from SJL mice immunized with PLP139-151 as described *(*Piccio L. et al., J. Immunol. 168, 1940-1949, 2002*).* Lymphocytes were treated with test compound at concentrations between 5-100µM for 1h, while control/untreated cells were treated with vehicle (DMSO). Results are expressed as percentage of inhibition over control (= 100%) ± S.D and are the mean of three-four experiments.

### Chemotaxis assay

PMNs and monocytes migration was assessed using 1 or 3-µm pore size transwells (Bio-Coat Becton-Dickinson), while lymphocytes by using 5-µm pore size transwells.

Cells were in RPMI1640, containing 10% heat-inactivated Fetal Calf Serum (FCS) and 20 mM HEPES, pH 7.3, at 2x10⁶/ml. 100 µl of cell suspension were added to the top well, and 600 µl of medium, containing chemoattractants and test compound, were added to the bottom well. Migration was evaluated after 2h for PMNs and monocytes and after 4h for lymphocytes. fMLP 10 nM (PMNs and monocytes) and MIP-3β 100 nM (lymphocytes) were utilized as chemoattractants. After fixation with 1.5% glutaraldehyde, migrated cells were counted by fluorescence-activated celll sorting using polystyrene beads (Polyscience) as an internal standard *(*Campbell J.J. et al. J. Cell. Biol. 134, 255-266, 1996*;* Laudanna C. et al. J. Biol. Chem. 273,46, 30306-30315, 1998*).*

Results are expressed as percentage of inhibition over control (= 100%), and are the mean of three experiments.

### Respiratory burst (H₂O₂ assay)

NADPH-oxidase activation was measured as induction of H₂O₂ release (derived from dismutation of O₂⁻) triggered in PMNs and monocytes by PMA (200 ng/ml).

Briefly, PMNs and monocytes were pretreated at 37°C for 30-60 min with test compound at different concentrations, and stimulated with phorbol 12-myristate 13-acetate (PMA, 200 ng/ml) dissolved in 0.5 ml of a standard reaction mixture. Release of H₂O₂ was evalauted in real time at time points of 5 min by using a microplate fluorescence reader (Perkin-Elmer). Results are expressed as nmoles of H₂O₂ released over time by 10⁶ cells. Shown are dose-response curves of inhibitory effect on H₂O₂ release over 60 min.

### In-vivo studies

### Active induction and clinical evaluation of EAE

Female SJUJ mice, 6-8 weeks of age, were obtained from Harlan-Nossan Italy.

Mice were immunized s.c. with 200 µg PLP139-151 in 0.1ml emulsion consisting of equal volumes of PBS and CFA (Difco Laboratories, Detroit, MI) and containing 6mg/ml of *Mycobacterium tuberculosis* H37Ra (Difco). To induce a severe disease, in some experiments 400 ng Pertussis toxin (PTX) was administered i.v. at days 0 and 2 *(*Constantin, G. et al. Eur. J. Immunol., 28, 3523-3529, 1998). Mice were scored for EAE according to the following scale: 0, no disease; 1, tail weakness; 2, paraparesis; 3, paraplegia; 4, paraplegia with forelimb weakness or paralysis; 5, moribund or dead animals (Brocke S. et al., Autoimmune disease models. Academic Press, San Diego, 1-4, 1994).

Test compound was administered daily at 60 mg/kg i.p. for 17 days starting from the day of immunization. Control animals received vehicle (DMSO).

### Results

### In vitro studies

### Effect of V-ATPase inhibitors on rapid integrin activation and chemotaxis in human leukocytes

Integrin activation was measured as induction of rapid adhesion triggered by the "classical" chemoattractant fMLP (100 nM) or by the CC chemokine MIP3β (CCL19, 1 µM). PMNs were triggered to adhere to fibrinogen (CR3 ligand); lymphocytes were triggered to adhere to ICAM-1 (LFA-1 ligand).

All the V-ATPase inhibitors (Table 1) were able to inhibit rapid adhesion in both PMNs and lymphocytes, in a concentration-related manner. Compound of example 2 (PCT/EP2005/051910) was the most potent compound with about 100 % inhibition at 5-10 µM concentration. Compound of Example 2 (PCT/EP2005/051910) was also very potent in inhibiting adhesion in monocytes, while Compound of Example 1 (PCT/EP2005/051908) was ineffective in these cells. Notably, the chemical structure of Compound of Example 1 (PCT/EP2005/051908) makes this compound rather susceptible to esterase action. As monocytes highly express non-specific cytosolic esterases, this likely explains the lack of effect of Compound of Example 1 (PCT/EP2005/051908) on monocyte functions.

Data clearly show that Compound of Example 2 (PCT/EP2005/051910) and Compound of Example 1 (PCT/EP2005/051908) efficiently inhibited chemotaxis in the three major leukocyte sub-types.

Inhibitions observed are clearly concentration-dependent supporting a specific pharmacological effect.

These data suggest that the V-ATPase may be critical target to block the two major cellular events (integrin action and directional motility) responsible of leukocyte recruitment during immune system function and inflammation.

### Effect of V-ATPase inhibitors on spontaneous adhesion of murine encephalitogenic T cells

ICAM-1 and VCAM-1 are adhesion molecules from the family of immunoglobulins and control the migration of lymphocytes through the endothelium during inflammatory responses. Encephalitogenic T cells are activated lymphocytes displaying spontaneous adhesion on ICAM-1 and VCAM-1 and have increased migration capacities in inflammation sites. Spontaneous adhesion of encephalitogenic lymphocytes was measured in adhesion assays on purified ICAM-1 and VCAM-1 as described in the method.

The results on six V-ATPase inhibitors (Table 2) show that treatment of cells with 5 µM of all compounds efficiently blocked spontaneous adhesion on ICAM-1, a ligand for LFA-1 integrin. Compound of Example 2 (PCT/EP2005/051910) was the most potent compound, with an outstanding ability to inhibit the spontaneous adhesion at a concentration of 250 nM. The inhibition observed on ICAM-1 at 5 µM concentration for the remaining compounds was between 47 and 78%, while the inhibition observed using 50 µM concentration was almost complete. Less inhibition was observed with all compounds in adhesion assays on VCAM-1, a ligand for VLA-4 integrin. As shown for ICAM-1, Compound of Example 2 (PCT/EP2005/051910) was the most potent compound. Compound of Example 1 (PCT/EP2005/051908) was also very efficient in blocking spontaneous adhesion to VCAM-1.

These results indicate that some compounds may have integrin-selective blocking effects, while others may have broad effects on integrin family.

Inhibition was clearly concentration-dependent in all the experiments supporting a specific pharmacological effect. All treated cells with V-ATPase inhibitors were trypan blue negative and showed no morphological alterations.

### Effect of V-ATPase inhibitors on chemokine-induced adhesion of murine encephalitogenic T cells

Chemokines trigger rapid arrest of lymphocytes in blood vessels through pertussis toxin-sensitive G protein coupled receptors. Rapid adhesion to purified ICAM-1 was measured in in vitro adhesion assays using control/untreated cells and lymphocytes treated with concentration between 5-50 µM for 1 h. Autoreactive lymphocytes were then stimulated with 1 µM CCL19 (MIP-3beta) for 3 min. CCL19 was shown to be expressed on vascular endothelium in lymphoid organs and in sites of inflammation, including inflamed brain endothelium. The results shown in Table 3 demonstrate an almost complete block of autoreactive lymphocyte adhesion with the following compounds: Compound of Example 2 (PCT/EP2005/051910), Compound of Example 1 (PCT/EP2005/051908), Compound of Example 17 (PCT/EP2005/051908), Compound of Example 27 (PCT/EP2005/ 051908), Compound of Example 3 (PCT/EP2005/051908) and Compound of Example 44 (PCT/EP2005/051908). All these compounds were already efficient in blocking adhesion using a concentration of 5 µM for the cell treatment.

However, Compound of Example 2 (PCT/EP2005/051910) presented the highest efficiency in blocking activated T cell adhesion; this compound was already extremely efficient at the concentration of 1 µM. Compound of Example 14 (PCT/EP2005/051908) and Compound of Example 76 (PCT/EP2005/051908) blocked adhesion with efficiency between 50-60 % using a concentration of 50 µM (Table 3).

All the other tested compounds inhibited chemokine-induced adhesion with less efficiency or had no significant effect on adhesion at the highest concentration tested (50 µM) when compared with the compounds mentioned above.

Inhibition was clearly concentration-dependent in all the experiments supporting a specific pharmacological effect. Flow cytometry experiments clearly showed that treatment of encephalitogenic T cells with all compounds does not modify the expression of adhesion molecules such as integrins, selectins, mucins and CD44 on cell surface, indicating that V-ATPase inhibitors block adhesion by interfering with signal transduction pathways controlling lymphocyte adhesion. These results, together with the data obtained in spontaneous adhesion assays using encephalitogenic lymphocytes, suggest that V-ATPase may represent critical target to block autoreactive T cell recruitment during autoimmune diseases.

### Effect of V-ATPase inhibitors on NADPH-oxidase activation

The data (Figure 1) clearly show that Compound of Example 2 (PCT/EP2005/051910) and Compound of Example 1 (PCT/EP2005/051908) inhibits NADPH-oxidase activation both in PMNs and in monocytes. Inhibition is concentration-dependent supporting a specific pharmacological effect.

Compound of Example 2 (PCT/EP2005/051910) showed a great potency in this model. The data suggest that V-ATPase may be a critical target to block the release of oxygen-derived free radicals, a recognized pro-inflammatory event during the innate immune response.

### In vivo studies

### Effect of a V-ATPase inhibitor on EAE

As Compound of Example 1 (PCT/EP2005/051908) blocked lymphocyte adhesive interactions implicated in the pathogenesis of EAE, the animal model of multiple sclerosis, we finally determined its effects on the disease (Table 4). SJL mice were immunized with PLP139-151 peptide from cerebral proteolipid. The disease model has relapses and remission periods, as described in relapsing-remitting MS, the most frequent form of human disease accounting for 85% of all MS patients. Mice received 60 mg/kg daily of Compound of Example 1 (PCT/EP2005/051908), intraperitoneally, for 17 days starting from the day of immunization. Control animals received vehicle (DMSO). Control animals developed disease with a mean day of onset of 13±1.5. Mean maximum clinical score in control animals was 2.7±0.3. All control mice that developed disease also presented clinical relapses. Compound of Example 1 (PCT/EP2005/051908)- treatment completely blocked the development of clinical signs of disease in all treated animals. No animals treated with Compound of Example 1 (PCT/EP2005/051908) developed clinical relapses during an observation period of 61 days. Administration of Compound of Example 1 (PCT/EP2005/051908) did not cause any visible toxicity itself, as assessed by the appearance of the treated animals or macroscopic pathological analysis (not shown).

The complete block of the in vivo EAE strongly support the therapeutic potential of this compound in multiple sclerosis and autoimmune diseases.

**TABLE 1**

| Compound | Structure | [cpd], µM | Inhibition of Adhesion (%) | | | Inhibition of Chemotaxis (%) | | |
|---|---|---|---|---|---|---|---|---|
| | | | PMNs (fMLP) | Lymphocytes (MIP3-β) | Monocytes (fMLP) | PMNs (fMLP) | Lymphocytes (MIP3-β) | Monocytes (fMLP) |
| Compound of Example 2 (**) | 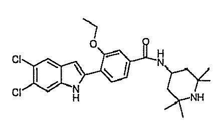 | 1 | 3.4 | 2.9 | 0 | 11.4 | 27.0 | 14.6 |
| | | 2.5 | 71.0 | 13.6 | 86.3 | 53.0 | 46.7 | 44.3 |
| | | 5 | 93.6 | 71.8 | 94.7 | 64.6 | 60.0 | 56.4 |
| | | 10 | 100 | 99.5 | 98.6 | 83.9 | 81.5 | 77.0 |
| | | 25 | 100 | 100 | 100 | 89.7 | 100 | 87.7 |
| | | 50 | 100 | 100 | 100 | 96.3 | 100 | 100 |
| | | 100 | 100 | 100 | 100 | 100 | 100 | 100 |
| Compound of Example 1(*) | 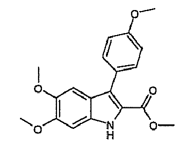 | 2.5 | 9.4 | 32.4 | <5 | 14.6 | 11.6 | 10.5 |
| | | 5 | 56.1 | 41.2 | <5 | 28.2 | 27.2 | 15.5 |
| | | 10 | 66.7 | 42.6 | <5 | 34.0 | 43.2 | 18.3 |
| | | 25 | 72.9 | 60.8 | <5 | 40.6 | 53.3 | 47.4 |
| | | 50 | 79.0 | 61.8 | <5 | 46.4 | 63.5 | 46.3 |
| | | 100 | 94.2 | 98.6 | <5 | 62.6 | 86.9 | 75.5 |
| Compound of Example14(*) | 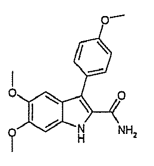 | 25 | 62.8 | 30.9 | | | | |
| | | 50 | 83.9 | 70.9 | | | | |
| | | 100 | 94.9 | 91.8 | | | | |
| Compound of Example26 (*) | 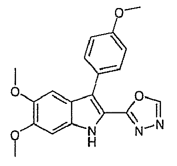 | 25 | 34.7 | 8.7 | | | | |
| | | 50 | 50.5 | 21.9 | | | | |
| | | 100 | 57.5 | 26.7 | | | | |
| Compound of Example 28(*) | 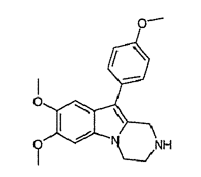 | 25 | 34.7 | 16.7 | | | | |
| | | 50 | 41.0 | 34.8 | | | | |
| | | 100 | 46.5 | 49.1 | | | | |
| Compound of Example39 (*) | 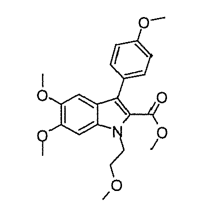 | 25 | 33.8 | 8.2 | | | | |
| | | 50 | 39.1 | 26.4 | | | | |
| | | 100 | 44.8 | 41.4 | | | | |
| Compound of Example 52 (*) | 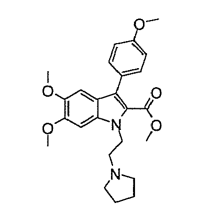 | 25 | 0.8 | 45.9 | | | | |
| | | 50 | 11.0 | 53.5 | | | | |
| | | 100 | 6.1 | 58.9 | | | | |
| Compound of Example 56 (*) | 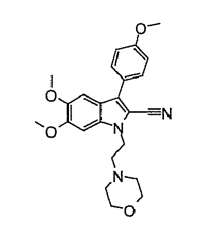 | 25 | 3.4 | 0 | | | | |
| | | 50 | 11.6 | 12.6 | | | | |
| | | 100 | 8.7 | 72.8 | | | | |
| Compound of Example 76 (*) | 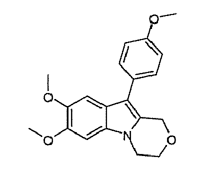 | 25 | 25.6 | 30.1 | | | | |
| | | 50 | 35.7 | 40.3 | | | | |
| | | 100 | 66.0 | 48.3 | | | | |
| Compound of Example 81 (*) | 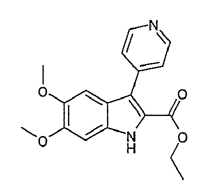 | 25 | 14.4 | 4.5 | | | | |
| | | 50 | 0.8 | 12.7 | | | | |
| | | 100 | 0 | 34.9 | | | | |

**TABLE 2**

| Compound | Structure | [cpd], µM | Inhibition of Spontaneous Adhesion in encephalitogenic lymphocytes (% of control/non-treated cells) | |
|---|---|---|---|---|
| | | | ICAM-1 | VCAM-1 |
| Compound of Example 2 (**) | 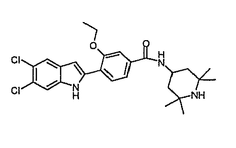 | 0.25 | 83.0 | 11.0 |
| | | 0.5 | 89.2 | 71.0 |
| | | 1 | 95.5 | 99.5 |
| | | 5 | 96.6 | 100 |
| | | 10 | 99.7 | 100 |
| Compound of Example 1 (*) | 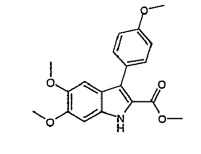 | 5 | 47.0 | 46.0 |
| | | 10 | 65.0 | 66.0 |
| | | 25 | 70.0 | 74.0 |
| | | 50 | 89.0 | 92.4 |
| | | 100 | 96.0 | n.d. |
| Compound of Example 17 (*) | 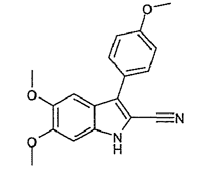 | 5 | 72.8 | 34.0 |
| | | 10 | 72.5 | 53.0 |
| | | 25 | 76.0 | 71.5 |
| | | 50 | 92.0 | 89.0 |
| | | 100 | 99.0 | n.d. |
| Compound of Example 27 (*) | 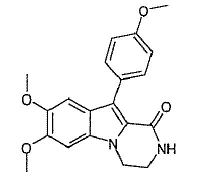 | 5 | 69.0 | 11.0 |
| | | 10 | 76.0 | 31.0 |
| | | 25 | 82.0 | 35.5 |
| | | 50 | 90.0 | 82.0 |
| | | 100 | 99.0 | n.d. |
| Compound of Example 3 (*) | 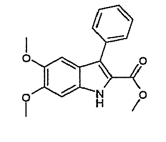 | 5 | 78.0 | 6.0 |
| | | 10 | 79.5 | 9.0 |
| | | 25 | 90.0 | 32.5 |
| | | 50 | 92.0 | 71.4 |
| | | 100 | 99.0 | n.d. |
| Compound of Example 44 (*) | 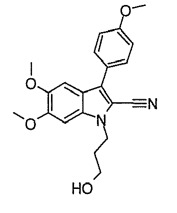 | 5 | 75.0 | 29.0 |
| | | 10 | 84.0 | 39.0 |
| | | 25 | 88.0 | 43.0 |
| | | 50 | 91.5 | 84.0 |
| | | 100 | 99.0 | n.d. |

**TABLE 3**

| Compound | Structure | [cpd], µM | Chemokine-induced Adhesion in encephalitogenic lymphocytes Mean counts ± S.D. | |
|---|---|---|---|---|
| | | | in absence of MIP3-β stimulation | in presence of MIP3-β stimulation |
| Compound of Example 2 (**) | 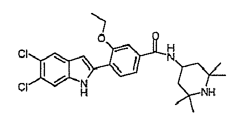 | NT | 29.0 ± 8.0 | 68.0±7.0 |
| | | 1 | 1.3±2.8 | 11.4±5.4 |
| | | 5 | 0.6±1.6 | 4.6±2.4 |
| | | 10 | 0.4±0.4 | 0.4±0.8 |
| Compound of Example 1 (*) | 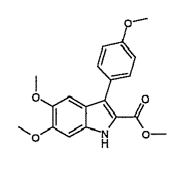 | NT | 29.0±8.0 | 68.0±7.0 |
| | | 5 | 1.3±1.0 | 10.3±1.0 |
| | | 10 | 1.0±1.0 | 7.0±1.0 |
| | | 25 | 0.4±0.2 | 5.3±2.0 |
| | | 50 | 0.4±0.1 | 3.2±1.0 |
| Compound of Example 17 (*) | 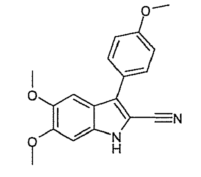 | NT | 29.0±8.0 | 68.0±7.0 |
| | | 5 | 2.0±1.0 | 13.6±3.5 |
| | | 10 | 0.9±0.2 | 10.0 ± 3.0 |
| | | 25 | 0.6±0.5 | 1.8 ± 1.0 |
| | | 50 | 0.4 ± 0.1 | 2.1 ±0.9 |
| Compound of Example 14 (*) | 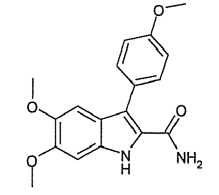 | NT | 90.9±5.3 | 140.0±3.6 |
| | | 1 | 67.6±2.0 | 120.3±9.8 |
| | | 5 | 41.8±4.2 | 101.2±6.1 |
| | | 50 | 20.9±11.7 | 56.8±2.2 |
| Compound of Example 26 (*) | 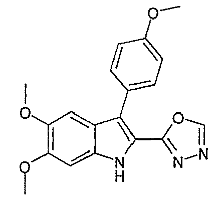 | NT | 90.9±5.3 | 140.0±3.6 |
| | | 5 | 93.1±7.5 | 125.9±7.2 |
| | | 50 | 49.8±5.9 | 98.9±12.0 |
| Compound of Example 27 (*) | 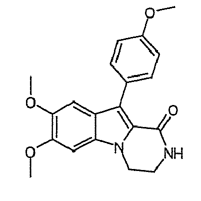 | NT | 29.0±8.0 | 68.0±7.0 |
| | | 5 | 5.0±2.2 | 17.7±2.5 |
| | | 10 | 1.7±1.0 | 18.1 ±4.3 |
| | | 25 | 1.5±0.1 | 9.3 ± 0.5 |
| | | 50 | 1.2±1.1 | 5.1 ±1.1 |
| Compound of Example 3 (*) | 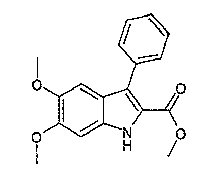 | NT | 29.0±8.0 | 68.0±7.0 |
| | | 5 | 1.5±.2.0 | 10.5 ± 3.0 |
| | | 10 | 1.8±1.0 | 8.7±3.0 |
| | | 25 | 1.2±1.0 | 3.5±1.5 |
| | | 50 | 0.5±2.0 | 5.0 ±2.0 |
| Compound of Example 28 (*) | 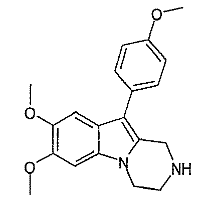 | NT | 90.9±5.3 | 140.0±3.6 |
| | | 5 | 75.7±9.0 | 127.0±5.0 |
| | | 50 | 48.6±2.9 | 118.8±10.8 |
| Compound of Example 39 (*) | 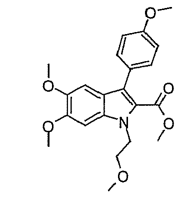 | NT | 90.9±5.3 | 140.0±3.6 |
| | | 5 | 72.9±8.8 | 118.2±10.0 |
| | | 50 | 44.8±1.9 | 104.4±5.0 |
| Compound of Example 50 (*) | 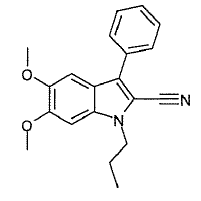 | NT | 90.9±5.3 | 140.0±3.6 |
| | | 5 | 81.7±11.9 | 124.5±8.1 |
| | | 50 | 62.0±4.0 | 92.0±3.0 |
| Compound of Example 52 (*) | 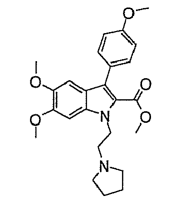 | NT | 90.9±5.3 | 140.0±3.6 |
| | | 1 | 51.9±3.9 | 118.9±4.0 |
| | | 5 | 31.8±3.1 | 103.2±10.2 |
| | | 50 | 24.9±12.8 | 82.3±8.1 |
| Compound of Example 44 (*) | 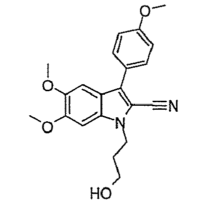 | NT | 29.0±8.0 | 68.0±7.0 |
| | | 5 | 1.0±0.9 | 2.1 ±0.5 |
| | | 10 | 0.4±1.7 | 3.7±1.1 |
| | | 25 | 0.4±0.1 | 1.8±0.5 |
| | | 50 | 0.4±0.5 | 0.1±0.5 |
| Compound of Example 56 (*) | 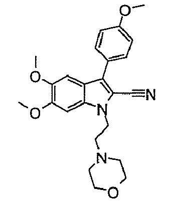 | NT | 90.9±5.3 | 140.0±3.6 |
| | | 5 | 64.1±9.9 | 92.3±2.3 |
| | | 50 | 49.7±3.9 | 79.4±12.1 |
| Compound of Example 76 (*) | 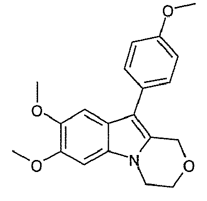 | NT | 90.9±5.3 | 140.0±3.6 |
| | | 1 | 36.9±4.0 | 102.9±2.9 |
| | | 5 | 28.3±8.9 | 87.7±12.0 |
| | | 50 | 23.0 ± 1.9 | 66.6 ± 1.8 |
| Compound of Example 68 (*) | 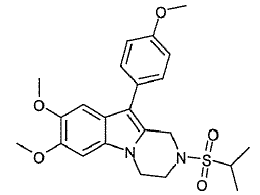 | NT | 90.9±5.3 | 140.0±3.6 |
| | | 5 | 62.3±9.0 | 102.8±5.5 |
| | | 50 | 33.5±6.0 | 74.3±3.9 |
| Compound of Example 81 (*) | 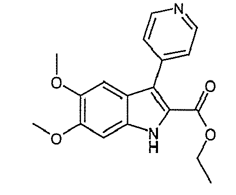 | NT | 90.9±5.3 | 140.0±3.6 |
| | | 5 | 56.2±11.1 | 84.5±8.8 |
| | | 50 | 41.3±3.6 | 99.2±5.0 |

**TABLE 4**

| | Animals number | Mortality | Disease incidence | Mean day of onset | Mean maximum clinical score |
|---|---|---|---|---|---|
| Control | 5 | 0/5 | 4/5 | 13.0 ± 1.5 | 2.7 ± 0.3 |
| Compound of Example 1 (*) 60 mg/kg i.p. | 6 | 0/6 | 0/6 | - | 0 |

| | | | | | |
|---|---|---|---|---|---|
| (*): PCT/EP2005/051908 (**):PCT/EP2005/051910 | | | | | |

## Claims

1. Use of one or more V-ATPase inhibitors in the preparation of a medicament useful for preventing and/or treating inflammatory and autoimmune diseases.

2. Use according to claim 1, wherein said V-ATPase inhibitor is selected from bafilomycins, concanamycins, depsipeptide mycotoxins derived from the fungi *Metharisium anisopliae*, destruxin, lobatamides, salicylihalamides, oximidines, and derivatives thereof.

3. Use according to claim 1, wherein said V-ATPase inhibitor is selected from sulphonamide derivatives of bafilomycins, 7,21-0-disubstituted bafilomycins, 2-methoxy-2,4-pentadienoic esters of bafilomycins,

4. Use according to claim 1 wherein said V-ATPase inhibitor is selected from N-ethylmaleimide, 7-chloro-4-nitrobenzo-2-oxa-1,3-diazole, WY 47766, SB242784, FR167356.

5. Use according to claim 1 wherein said V-ATPase inhibitor is a 2- and/or 3-substituted indole, a 2- and/or 3- substituted azaindole or a 2- and/or 3-substituted benzimidazole.

6. Use according to claims 1-6, wherein said inflammatory disease is comprised within the group of rheumatoid arthritis, atherosclerosis, multiple sclerosis, lung fibrosis, life-threatening hypersensitivity (anaphylaxis), reactions to insect bites, drugs, and toxins; chronic inflammatory bowel diseases, such as irritable bowel syndrome, colitis and other inflammatory diseases of the gastrointestinal tract; nephritis; inflammatory skin diseases like eczema, dermatitis, ichthyosis, acne, skin hypersensitivity reactions.

7. Use according to claims 1-6, wherein said autoimmune disease is comprised within the group of type 1 diabetes, systemic lupus erythematosus, multiple sclerosis, rheumatoid arthritis, inflammatory bowel diseases, including both Crohn's disease and ulcerative colitis, hemolytic anemia, Graves' disease, scleroderma, autoimmune thyroid diseases, Sjögren's syndrome, psoriasis, eye autoimmune diseases, myasthenia gravis, Guillain-Barre' syndrome and Addison's disease.

8. Use according to claims 1-7, wherein said V-ATPase inhibitor is administered in the form of pharmaceutical compositions in the presence of suitable pharmaceutical excipients and possible additional drugs useful in antiinflammatory / autoimmune therapy.

9. Use according to claim 8, wherein said pharmaceutical composition contain said V-ATPase inhibitor in a quantity between 1 and 1000 mg.

10. Use according to claims 9, wherein said composition is adapted to deliver to the patient a dosage comprised between 0.01 and 100 mg/Kg.

11. Use according to claims 8-10, wherein said pharmaceutical composition is suitable for oral, buccal, intravenous, intramuscular, intradermic, transdermal, topic, ophthalmic, intraauricular or inhalatory administration route.

12. Use according to claims 8-11, wherein said pharmaceutical composition is provided in the form of injectable solutions, solutions for infusion, solutions for inhalation, suspensions, emulsions, syrups, elixirs, drops, suppositories, possibly coated pills, hard or soft capsules, microcapsules, granules, dispersible powders.
